# EUROPEAN PATENT APPLICATION

(11) **EP 4 053 853 A1**
(43) Date of publication of application: **07.09.2022**
(21) Application number: 21159970.9
(22) Date of filing: 01.03.2021
(51) Int. Cl.: G16H 50/20

(54) **APPARATUS FOR DETERMINING A TEMPORAL BREAST CANCER RISK**

(71) Applicant: ScreenPoint Medical B.V., 6525 Nijmegen (NL)
(72) Inventor: Karssemeijer, Nico, 6525 NIJMEGEN (NL); Dalmis, Mehmet, 6525 NIJMEGEN (NL); Rodriguez, Alejandro, 6525 NIJMEGEN (NL); Jansen, Natasja, 6525 NIJMEGEN (NL); Kallenberg, Michiel, 6525 NIJMEGEN (NL); Schouwenberg, Jeroen, 6525 NIJMEGEN (NL)
(74) Representative: Eisenführ Speiser

(57) **Abstract**

The invention relates to an apparatus for determining a temporal breast cancer risk (27). This risk is indicative of detecting cancer in a breast within a predetermined time interval after a negative breast cancer detection result that has been obtained by carrying out a breast cancer detection procedure defined by a breast cancer detection parameter. The temporal breast cancer risk (27) is determined based on a) a probability parameter (25) being indicative of a probability of having breast cancer and b) a density parameter (26) being indicative of a density-related property of breast tissue of a breast, wherein the probability parameter (25) and the density parameter (26) have been determined based on a breast image data set (24) of the woman by using an artificial intelligence. The determined risk can be used, for instance, for deciding whether a supplemental examination should be performed immediately or for defining a screening interval.

## Description

### FIELD OF THE INVENTION

The invention relates to an apparatus, a method and a computer program for determining a temporal breast cancer risk. The invention relates further to a training apparatus, a training method and a training computer program for training an artificial intelligence to be used by the apparatus, the method and the computer program for determining the temporal breast cancer risk.

### BACKGROUND OF THE INVENTION

A positive effect of breast cancer screening on mortality has been demonstrated in reviews of large population based programs. Breast cancer mortality reductions between 24% and 48% have been reported among women who regularly attend screening. However, despite these clear benefits, it remains the case that a substantial number of women, even though they perfectly comply with screening protocols, die from breast cancer. Approximately 30% of breast cancers are detected in-between screenings (interval cancers) and 25-30% of screen-detected cancers are retrospectively detectable on previous mammograms, and thus could have been detected earlier. Evidently, there is a clear need for improved breast cancer screening.

The major shortcoming of current day breast cancer screening is the one-size-fits-all approach that is currently applied for the majority of women in a specific age range; they all undergo the same screening protocol, with the same diagnostic modality, digital mammography (DM) or digital breast tomosynthesis (DBT), and with the same screening interval. It would therefore be helpful, if high quality guidance for a woman-specific adaptation of the screening procedure could be provided, in order to, for instance, adapt the screening interval and/or suggest an additional screening examination for a specific woman, for example a supplemental magnetic resonance imaging (MRI) or ultrasound (US) examination.

### SUMMARY OF THE INVENTION

It is an object of the present invention to provide an apparatus, a method and a computer program for determining temporal breast cancer risk being indicative of detecting cancer in a breast of a woman within a predetermined time interval after a negative breast cancer detection result, in order to provide guidance for a personalized adaptation of a screening procedure. For instance, the determined temporal breast cancer risk can be used for deciding whether immediately a supplemental screening examination, i.e. a supplemental breast cancer detection examination, should be carried out, and, if so, which kind of supplemental screening examination should be carried out. The determined temporal breast cancer risk can also be used for modifying a screening interval. If the determined temporal breast cancer risk indicates that breast cancer will relatively likely be detected within the predetermined time interval, an initially planned screening interval might be reduced, wherein otherwise it might not be modified or it could be increased. It is a further object of the present invention to provide a training apparatus, a training method and a computer program for training an artificial intelligence to be used by the apparatus, the method and the computer program for determining the temporal breast cancer risk.

In a first aspect of the present invention an apparatus for determining a temporal breast cancer risk as defined by claim 1 is presented. The temporal breast cancer risk is indicative of detecting cancer in a breast of a woman within a predetermined time interval, after a negative breast cancer detection result obtained by carrying out a breast cancer detection procedure defined by a breast cancer detection parameter. The apparatus comprises:
- a parameter providing module configured to provide a) a probability parameter, for the woman, being indicative of a probability of having breast cancer and b) a density parameter being indicative of a density-related property of breast tissue of a breast of the woman, wherein the probability parameter and the density parameter have been determined based on a breast image data set of the woman,
- an artificial intelligence providing module configured to provide an artificial intelligence which has been trained to provide, as an output, a temporal breast cancer risk being indicative of a risk of detecting cancer in a breast of a woman within a predetermined time interval after a negative breast cancer detection result obtained by carrying out a breast cancer detection procedure defined by the breast cancer detection parameter, wherein the artificial intelligence has been provided, as an input, with a) a probability parameter, for the woman, being indicative of a probability of having breast cancer and b) a density parameter being indicative of a density-related property of breast tissue of a breast of the woman, wherein the probability parameter and the density parameter have been determined based on a breast image data set of the woman,
- a temporal breast cancer risk determination module configured to determine the temporal breast cancer risk based on the provided probability parameter and the provided density parameter by using the provided artificial intelligence.

It has been found that a temporal breast cancer risk being indicative of detecting cancer in a breast of a woman within a predetermined time interval after a negative breast cancer detection result can be very reliably determined, if an artificial intelligence is used which is trained to output this temporal breast cancer risk based on, as input, a probability parameter being indicative of a probability of having breast cancer and a density parameter being indicative of a density-related property of breast tissue of a breast of a woman, wherein the probability parameter and the density parameter have been determined based on a breast image data set of the woman. Moreover, since this high-quality temporal breast cancer risk determination can be carried out by using an artificial intelligence only having a relatively small amount of input data, the artificial intelligence can be trained with relatively low training efforts. For instance, in an embodiment it can only have the provided probability parameter and the provided density parameter as input data.

The determined woman-specific high-quality temporal breast cancer risk, which is indicative of detecting breast cancer within a predetermined time interval after a negative breast cancer detection result, can be used for deciding whether, for instance, a supplemental breast cancer screening examination should be carried out immediately, or for defining a screening interval. If the determined temporal breast cancer risk is larger, the screening interval might be smaller, and, if the determined temporal breast cancer risk is smaller, the screening interval might be larger, i.e. the screening interval can be decreased or increased in a personalized manner. Since the determined woman-specific temporal breast cancer risk can also be used to enlarge a screening interval, i.e. to enlarge the time period between two consecutive breast cancer screening dates, it can also be used to reduce the x-ray dose which is generally applied during a breast cancer screening examination, because then over the lifetime of the woman less breast cancer screening examinations are required.

The determined breast cancer risk is called "temporal", because it is indicative of detecting cancer in a breast of a woman within a predetermined time interval, after a negative breast cancer detection result, i.e. it does not indicate the risk of detecting breast cancer any time in the future, but it indicates the risk of detecting breast cancer within a certain time after a negative screening result.

The breast cancer detection parameter defines the breast cancer detection procedure which is used for providing a positive or negative breast cancer detection result. In order to define a respective breast cancer detection procedure, the breast cancer detection parameter is indicative of at least one property of the breast cancer detection procedure. For instance, the breast cancer detection parameter can define which kind of measurement is carried out while performing the breast cancer detection procedure, i.e., for instance, what kind of breast image data set is used for the breast cancer detection procedure. The breast cancer detection parameter can also be indicative of a statistical property of the breast cancer detection procedure. For instance, it can be indicative of a recall rate and/or a detection rate of the breast cancer detection procedure.

The artificial intelligence providing module is configured to provide an artificial intelligence which has been trained to provide, as an about, a temporal breast cancer risk being indicative of a risk of detecting cancer in a breast of a woman within a predetermined time interval after a negative breast cancer detection result obtained by carrying out a breast cancer detection procedure defined by the breast cancer detection parameter, i.e. defined by the same breast cancer detection parameter which defines the breast cancer detection procedure which led to a current negative breast cancer detection result for a woman for which currently the temporal breast cancer risk should be determined. It is therefore ensured that the training of the artificial intelligence was based on a breast cancer detection procedure which is similarto the breast cancer detection procedure, which led to the current negative breast cancer detection result, at least with respect to the property of the breast cancer detection procedure for which the breast cancer detection parameter is indicative. This property can be, as explained above, a measurement property like the type of breast image data set used for the breast cancer detection and/or a statistical property. It is hereby noted that the expression "A and/or B" means "A without B" or "B without A" or "A and B", i.e., for instance, the breast cancer detection parameter can be indicative of a) a measurement property, but not for a statistical property, or b) a statistical property, but not for a measurement property, or c) a statistical property and a measurement property. In particular, in an embodiment the breast cancer detection parameter can include one or several measurement properties, which especially define the type of measurement, and/or one or several statistical properties.

The probability parameter is indicative of the probability of having breast cancer now, whereas the temporal breast cancer risk is indicative of the likelihood that within the predetermined time interval breast cancer will be detected, although currently it has not been detected. The temporal breast cancer risk does therefore not indicate whether currently breast cancer is present or not, but it refers to the risk that breast cancer will be detected within the predetermined time interval after a negative breast cancer screening, wherein this might include that at the time of breast cancer screening breast cancer was already present but not detected or that breast cancer was indeed not present.

The trained artificial intelligence, which is trained to output the temporal breast cancer risk based on the probability parameter and the density parameter, can be, for instance, based on a decision tree, a neural network or another machine learning architecture. In an embodiment the artificial intelligence comprises a random forest classifier for determining the temporal breast cancer risk. If the artificial intelligence uses a neural network, preferentially it does not use a convolutional neural network, particularly not a deep convolutional neural network, because due to the relatively low amount of input data such a more complicated artificial intelligence might not be necessarily required. However, it is nevertheless possible that the artificial intelligence comprises a convolutional network, particularly a deep convolutional network. The artificial intelligence could also be regarded as being an artificial intelligence module or unit.

The parameter providing module preferentially is configured to provide, as the density parameter, i) a dense density parameter being indicative of an amount of dense breast tissue of the breast having a density being larger than the density of other breast tissue of the breast, and/or ii) a density distribution parameter being indicative of the density distribution in the breast. The breast comprises glandular, connective and fat tissue, wherein the dense breast tissue is preferentially glandular and connective tissue or non-fat tissue. Thus, preferentially the parameter providing module is configured to provide, as the dense density parameter, a parameter being indicative of an amount of glandular and connective tissue of the breast as the dense breast tissue having a density being larger than the density of the fat tissue of the breast. This amount is preferentially a relative amount, i.e. it is the amount of the glandular and connective tissue relative to the entire breast or relative to the amount of fat tissue of the breast. The amount can be a volume amount, particularly if the breast image data set is suitable to determine volumes of tissues, or an area amount, particularly if the breast image data set includes a two-dimensional image of the breast, wherein the area amount can refer the total area of breast image, which shows glandular and connective tissue. Again, the volume amount or area amount is preferentially a relative amount. It has been found that, by using these parameters as input for the artificial intelligence, the quality, i.e. the accuracy, of determining the temporal breast cancer risk can be further improved.

Preferentially, the apparatus further comprises a breast image data set providing module configured to provide a breast image data set of a breast of the woman, wherein the parameter providing module is configured to determine the probability parameter and/or the density parameter based on the provided breast image data set and to provide the determined probability parameter and/or the determined the density parameter, respectively. In an embodiment, the parameter providing module is configured to determine the probability parameter by using a further artificial intelligence. In particular, the parameter providing unit can be configured to determine the probability parameter by using the Transpara module of the company ScreenPoint Medical. However, the parameter providing module can also be configured to use another software product for determining the probability parameter like known computer-aided detection (CAD) software products which provide a probability parameter being indicative of a probability of having breast cancer.

For determining the dense density parameter, in an embodiment, the parameter providing module might comprise the LIBRA software of the Laboratory of Individualized Radiosensitiy Assessment (LIBRA). The parameter providing unit can also determine the parameters in other way. For instance, for determining the dense density parameter also the technique can be used, which is described in the article "Volumetric Breast Density Estimation From Full-Field Digital Mammograms" by S. van Engeland et al., IEEE Transactions on Medical Imaging, volume 25, pages 273 to 282 (2006), which is herewith incorporated by reference.

The density distribution parameter, which might also be regarded as being a tissue texture parameter, is indicative of the density distribution in the breast. For instance, it can be indicative of the degree of homogeneity of the distribution of the different types of breast tissue having different densities within the respective breast, and/or it can be indicative of a variation of the amount of dense breast tissue in different quadrants or sections of the breast. For instance, the breast can be subdivided into sections, wherein for each section a dense density parameter can be determined, which is indicative of the amount of dense breast tissue of the breast having a dense density being lager than the density of other breast tissue of the breast in the respective section. In particular, the volume or area percentage of glandular and connective tissue within a respective section can be determined. The density distribution parameter then can be indicative of the distribution of the different dense density parameters determined for the different sections. In particular, the density distribution parameter can be indicative of the variation of this distribution of dense density parameters determined for the different sections of the breast. In an embodiment, the density distribution parameter can be determined by using the technique disclosed in the article "Unsupervised deep learning applied to breast density segmentation and mammographic risk scoring" by M. Kallenberg et al., IEEE Transactions on Medical Imaging, volume 35, pages 1322 to 1331 (2016), which is herewith incorporated by reference.

The breast image data set can include one or several two-dimensional images of the breast and/or one or several three-dimensional images of the breast. Moreover, the breast image data set can include at least one of a mammography image and a DBT image. The mammography image can have been acquired by using an x-ray film and digitizing the x-ray film, wherein this could be named classical mammography, or the mammography image could have been acquired by using solid-state detectors directly producing a digital mammography (DM) image. In a preferred embodiment, the breast image data set includes a DBT image and a DM image.

Although above it is described that the parameter providing module is configured to determine the parameters, the parameter providing module can also just be a receiving module configured to receive the respective parameter from another module, which determines the respective parameter, and provide the received respective parameter. Moreover, the parameter providing module can also be a storage in which a determined parameter is stored and from which it is retrieved for providing the stored parameter.

In an embodiment, the artificial intelligence providing module is configured to determine the artificial intelligence by training as described further below with respect to a training apparatus, i.e. the artificial intelligence providing module can be such a training apparatus. However, the artificial intelligence providing module can also just be a receiving module configured to receive the artificial intelligence from another module, which, for instance, determines the artificial intelligence by training, and provide the received artificial intelligence. Moreover, the artificial intelligence providing module can also be a storage in which the already trained artificial intelligence is stored and from which it is retrieved for providing the stored artificial intelligence.

In a preferred embodiment, i) the parameter providing module is configured to also provide a density-distribution-based breast cancer risk, wherein the density-distribution-based breast cancer risk has been determined based on a density distribution parameter which is indicative of the density distribution in the breast tissue and which has been determined based on the breast image data set of the breast, ii) the artificial intelligence providing module is configured to provide, as the artificial intelligence, an artificial intelligence which has been trained to provide, as the output, the temporal breast cancer risk, after the artificial intelligence has also been provided, as the input, with a density-distribution-based breast cancer risk, wherein the density-distribution-based breast cancer risk has been determined based on a density distribution parameter which is indicative of the density distribution in the breast and which has been determined based on a breast image data set of the breast, and iii) the temporal breast cancer risk determination module is configured to determine the temporal breast cancer risk also based on the provided density-distribution-based breast cancer risk by using the provided artificial intelligence. In this embodiment, preferentially the parameter providing module is configured to determine the density distribution parameter based on the provided breast image data set of the breast and to determine the density-distribution-based breast cancer risk based on the determined density distribution parameter. For determining the density-distribution-based breast cancer risk known techniques can be used like the technique disclosed in the above mentioned article by M. Kallenberg et al. It has been found that, if also the density-distribution-based breast cancer risk is used for determining the temporal breast cancer risk, the temporal breast cancer risk can be determined even more accurately and hence with a further increased quality.

The density-distribution-based breast cancer risk is preferentially indicative of the likelihood that breast cancer is currently present, although it has not been detected, or will develop any time in the future. The parameter providing module can be configured to use a further artificial intelligence, particularly machine learning, for determining the density-distribution-based breast cancer risk, wherein this artificial intelligence preferentially is trained by using density distribution parameters which have been determined based on breast image data sets in which breast cancer was not detected and by using the information whether later breast cancer was detected or not. Thus, in an embodiment, the density-distribution-based breast cancer risk might be determined only based on the type of breast, i.e. based on the distribution of the three breast tissue types (glandular, connective and fat) in the breast.

In an embodiment, the artificial intelligence providing module and the temporal breast cancer risk determination module are configured such that the risk of getting breast cancer within a predetermined time interval being equal to or smaller than two years after the negative breast cancer detection result is determined as the temporal breast cancer risk. Moreover, in an embodiment, the artificial intelligence providing module and the temporal breast cancer risk determination module are configured such that the risk of getting breast cancer within a predetermined time interval being equal to or smaller than one year after the negative breast cancer detection result is determined as the temporal breast cancer risk.

In an embodiment, i) the parameter providing module is configured to provide at least one further parameter extracted from a dense breast tissue thickness map, ii) the artificial intelligence providing module is configured to provide, as the artificial intelligence, an artificial intelligence which has been trained to provide, as the output, the temporal breast cancer risk, after the artificial intelligence has also been provided, as the input, with the at least one further parameter, and iii) the temporal breast cancer risk determination module is configured to determine the temporal breast cancer risk also based on the provided further parameter by using the provided artificial intelligence. It has been found that by using at least one of these additional parameters the accuracy of the determined temporal breast cancer risk can be further improved.

The breast density thickness map is preferentially defined for a projection x-ray mammography image, wherein for each image element of the mammography image the thickness of dense breast tissue along the respective projection ray is determined, wherein the determined thickness value is then assigned to the image element, in order to generate the breast density thickness map. Thus, the breast density thickness can be regarded as being a kind of image, wherein each image element indicates the thickness of the dense breast tissue along the respective projection ray. The thickness value is preferentially the thickness of the glandular and connective tissue. In an embodiment, the dense breast tissue of which the thickness is determined is any non-fat tissue type. The breast density thickness map and the determination of the breast density thickness map is known, wherein these known maps and techniques for generating the maps can be used. For instance, the breast density thickness maps disclosed in the above mentioned article by S. van Engeland et al. and in the article "Quantification of masking risk in screening mammography with volumetric breast density maps" by K. Holland et al., Springer, Breast Cancer Research and Treatment, pages 1 to 8 (2017), which is also herewith incorporated by reference.

As a parameter extracted from a breast density thickness map a percentage dense area (PDA) could be used, which is indicative of the percentage area on the breast density thickness map where the dense tissue thickness exceeds a predefined thickness value like 1 cm. This parameter PDA is also defined in the above mentioned article by K. Holland et al. Alternatively or in addition, also a dense tissue masking model (DTMM) parameter could be extracted from the dense breast tissue thickness map and used for determining the temporal breast cancer risk. The DTMM parameter is known and described, for instance, in the above mentioned article by K. Holland et al., wherein in this regard it is particularly referred to the appendix of the article by K. Holland et al., because in the appendix a calculation of the DTMM parameter is described in detail.

In an embodiment, i) the artificial intelligence providing module is configured to provide the artificial intelligence such that it is trained to provide, as an output, temporal breast cancer risks for different types of cancer, wherein a respective temporal breast cancer risk is indicative of a risk of detecting cancer of the respective cancer type in the breast within the predetermined time interval after a negative breast cancer detection result obtained by carrying out a breast cancer detection procedure defined by the breast cancer detection parameter, wherein the artificial intelligence has been provided, as the input, with the probability parameter and the density parameter, and ii) the temporal breast cancer risk determination module is configured to determine the temporal breast cancer risks for the different cancer types based on the provided probability parameter and the provided density parameter by using the provided artificial intelligence. In particular, i) the parameter providing module can be configured to provide different probability parameters for the different cancer types being indicative of a probability of having breast cancer of a respective cancer type, b) the artificial intelligence providing module is configured to provide the artificial intelligence such that it is trained to provide, as an output, temporal breast cancer risks for different types of cancer, wherein a respective temporal breast cancer risk is indicative of a risk of detecting cancer of the respective cancer type in the breast within the predetermined time interval after a negative breast cancer detection result obtained by carrying out a breast cancer detection procedure defined by the breast cancer detection parameter, wherein the artificial intelligence has been provided, as the input, with different probability parameters for the different cancer types being indicative of a probability of having breast cancer of a respective cancer type and the density parameter, and iii) the temporal breast cancer risk determination module is configured to determine the temporal breast cancer risks for the different cancer types based on the provided probability parameters and the provided density parameter by using the provided artificial intelligence. The apparatus is especially configured to determine the temporal breast cancer risk for aggressive cancer types. Also in this embodiment, which is able to determine cancer-type-specific temporal breast cancer risks, the above described further parameters can be used.

This allows to provide a very differentiated temporal breast cancer risk, wherein for each of a given set of cancer types a respective risk of detecting cancer in the breast within the predetermined time interval after a negative breast cancer detection result can be provided. This can allow for a further improved guidance in determining which further breast cancer detection measurement should be carried out at which date.

In an embodiment, i) the artificial intelligence providing module is configured to provide the artificial intelligence such that it is trained to provide, as an output, temporal breast cancer risks for different predetermined time intervals, wherein a respective temporal breast cancer risk is indicative of a risk of detecting cancer in the breast within a respective predetermined time interval after a negative breast cancer detection result obtained by carrying out a breast cancer detection procedure defined by the breast cancer detection parameter, wherein the artificial intelligence has been provided, as the input, with the probability parameter and the density parameter, ii) the temporal breast cancer risk determination module is configured to determine the temporal breast cancer risks for the different predetermined time intervals based on the provided probability parameter and the provided density parameter by using the provided artificial intelligence. For instance, a set of temporal breast cancer risks can be determined for the different predetermined time intervals, which describe the risk of detecting cancer in the breast within one year, within two years, within three years, et cetera after a negative breast cancer detection result. This temporally further differentiated risk of detecting cancer allows for a further improved guidance in determining which further breast cancer detection measurement should be carried out at which date.

The apparatus can be configured to determine the temporal breast cancer risk per breast of a woman or for both breasts of the woman, wherein the latter refers to the risk that in any of the two breasts cancer will be identified in the predetermined time interval. In particular, a) the parameter providing module can be configured to provide a respective probability parameter and a respective density parameter for each breast of the woman, wherein the probability parameters and the density parameters have been determined based on the breast image data set which shows both breasts of the woman, b) the artificial intelligence providing module can be configured to provide, as the artificial intelligence, an artificial intelligence which has been trained to provide, as an output, i) a respective temporal breast cancer risk being indicative of a risk of detecting cancer in a respective breast of a woman within a predetermined time interval after a negative breast cancer detection result obtained by carrying out a breast cancer detection procedure defined by the breast cancer detection parameter and/or ii) a common temporal breast cancer risk being indicative of a risk of detecting cancer in any of the two breasts of a woman within a predetermined time interval after a negative breast cancer detection result obtained by carrying out a breast cancer detection procedure defined by the breast cancer detection parameter, wherein the artificial intelligence has been provided, as an input, with i) respective probability parameters being indicative of a respective probability of having breast cancer in a respective breast of the woman and ii) respective density parameters being indicative of a density-related property of breast tissue of the respective breast of the woman, wherein the probability parameters and the density parameters have been determined based on a breast image data set of the woman, and c) the temporal breast cancer risk determination module can be configured to determine the temporal breast cancer risk as i) a respective temporal breast cancer risk being indicative of a risk of detecting cancer in the respective breast of the woman within the predetermined time interval after the negative breast cancer detection result obtained by carrying out a breast cancer detection procedure defined by the breast cancer detection parameter and/or ii) a common temporal breast cancer risk being indicative of a risk of detecting cancer in any of the two breasts of the woman within the predetermined time interval after a negative breast cancer detection result, obtained by carrying out a breast cancer detection procedure defined by the breast cancer detection parameter, based on the provided probability parameters and the provided density parameters by using the provided artificial intelligence.

Preferentially, the artificial intelligence providing module and the temporal breast cancer risk determination module are configured such that the determined temporal breast cancer risk is a relative risk, which is indicative of the risk of detecting cancer for the respective woman within the predetermined time interval after a negative breast cancer detection result relative, obtained by carrying out a breast cancer detection procedure defined by the breast cancer detection parameter, to a risk of detecting cancer for a reference group of women within the predetermined time interval after a negative breast cancer detection result obtained by carrying out a breast cancer detection procedure defined by the breast cancer detection parameter. Thus, the determined temporal breast cancer risk can be indicative of whether the respective woman has an increased or decreased likelihood of detecting breast cancer within the predetermined time interval, wherein also the amount of increase or decrease can be indicated. This can allow for a further improved guidance in determining which breast cancer detection measurement should be carried out at which date.

In an embodiment the artificial intelligence providing module is configured to have several artificial intelligences for different breast cancer detection parameters and hence for different breast cancer detection procedures, wherein the apparatus comprises an input unit for allowing a user to indicate a certain breast cancer detection parameter and hence a certain breast cancer detection procedure, wherein the artificial intelligence providing module is configured to provide the artificial intelligence which corresponds to the indicated certain breast cancer detection procedure. This allows a user to adapt the apparatus for determining the temporal breast cancer risk to the respective breast cancer detection procedure which led to the negative breast cancer detection result.

In an embodiment the apparatus can also comprise the input for allowing a user to indicate a desired predetermined time interval. This allows to adapt the apparatus for determining the temporal breast cancer risk to, for instance, a desired screening interval which might be one year, two years, three years, et cetera.

The artificial intelligence providing module can also be configured to have several artificial intelligences for different woman-specific parameters being indicative for the specific woman for which the temporal breast cancer risk should be determined, wherein the apparatus can comprise an input unit for allowing the user to indicate a certain woman-specific parameter and wherein the artificial intelligence providing module is configured to provide the artificial intelligence which corresponds to the indicated woman-specific parameter. Thus, for different woman-specific parameters different artificial intelligences can be present, wherein for determining the temporal breast cancer risk for a specific woman the artificial intelligence is selected, which corresponds to, i.e. which is associated with, the indicated woman-specific parameter. The woman-specific parameter can be a parameter which characterizes the woman like her age, her body mass index, her personal history, genetic information, et cetera. The woman-specific parameter can also describe a statistical property of the population in which the woman is living like the prevalence of cancer in the population in which the woman is living. This allows to provide an artificial intelligence for determining the temporal breast cancer risk, which better fits to the respective woman, thereby allowing for a further improved determination of the temporal breast cancer risk.

In a further aspect of the present invention a training apparatus for training an artificial intelligence to be used by an apparatus for determining a temporal breast cancer risk as defined by any of claims 1 to 10 is presented, wherein the training apparatus comprises:
- a training data sets providing unit configured to provide training data sets, wherein each training data set includes, as input data, i) a probability parameter, for a woman, being indicative of a probability of having breast cancer and ii) a density parameter being indicative of a density-related property of breast tissue of a breast of the woman, and, as output data, a temporal breast cancer risk being indicative of a risk of detecting cancer in a breast within a predetermined time interval after a negative breast cancer detection result obtained by carrying out a breast cancer detection procedure defined by the breast cancer detection parameter,
- an artificial intelligence providing module configured to provide an artificial intelligence to be trained,
- a training module configured to train the provided artificial intelligence by using the provided training data sets such that deviations between i) outputs of the artificial intelligence, which are output by the artificial intelligence if the inputs of the training data sets are input into the artificial intelligence, and ii) outputs of the training data sets are minimized.

In a preferred embodiment, the training data sets are determined based on breast image data sets of breasts of women, in which breast cancer was not identified at screening and in which breast cancer was identified within the predetermined time interval after screening, for instance, within one or two years after screening. The breast image data sets, which are preferentially used for determining the training data sets, are acquired preferentially during the screening. Preferentially, for determining the training data sets also breast image data sets of breasts of women are used, in which breast cancer was not identified at screening and in which breast cancer was also not identified within the predetermined time interval after screening, for instance, within one or two years after screening. The breast image data sets can be used for determining the training input data, i.e. the probability parameter and the density parameter, and the training output data, i.e. the temporal breast cancer risk.

The training output data, i.e. the temporal breast cancer risk to be used during training, for a certain combination of the probability parameter and the density parameter, can be statistically determined based on several breast image data sets of several women for which a same respective probability parameter and a same respective density parameter have been determined. Thus, for a same combination of training input data several women are considered, wherein for each woman it is known whether cancer has been detected or has not been detected within the predetermined time interval after the negative breast cancer screening result. These several cancer detection and non-cancer detection events for a same combination of training input data can be used for determining the temporal breast cancer risk for this same combination of training input data, i.e. for determining the risk of detecting cancer within the predetermined time interval after a negative breast cancer screening. For instance, the training temporal breast cancer risk for the same combination of training input data can be determined as the number of cancer events divided by the total number of events for this combination of training input data. The temporal breast cancer risk can also be determined as a relative risk by dividing the number of cancer events by the number of non-cancer events for the respective combination of training input data. Generally, the likelihood of detecting cancer within the predetermined time interval might be divided by the likelihood of not detecting cancer within the predetermined time interval for determining the relative risk.

It is noted that the term "same" with respect to determining a training temporal breast cancer risk for a same combination of training input data depends on the desired accuracy of the probability parameter and of the density parameter for which a temporal breast cancer risk should be determined. For instance, if the probability parameter should be considered during training with an accuracy of 0.01, wherein the probability parameter ranges from 0.00 to 1.00 in this example, probability parameters which differ by, for instance, 0.001 would be regarded as being the same probability parameter, whereas probability parameters differing by 0.01 would be regarded as being different probability parameters. Correspondingly, also with respect to the density parameter the meaning of the term "same" of course depends on the accuracy of the density parameter which should be used during the training.

Also other techniques can be used for determining the training temporal breast cancer risk depending on a provided set of training probability parameters, training density parameters and the knowledge whether for the respective woman cancer has been detected within the predetermined time interval after a negative breast cancer screening result.

In another aspect of the present invention a method for determining a temporal breast cancer risk being indicative of detecting cancer in a breast of a woman within a predetermined time interval after a negative breast cancer detection result obtained by carrying out a breast cancer detection procedure defined by a breast cancer detection parameter is presented, wherein the method comprises:
- providing a) a probability parameter, for the woman, being indicative of a probability of having breast cancer and b) a density parameter being indicative of a density-related property of breast tissue of a breast of the woman, wherein the probability parameter and the density parameter have been determined based on a breast image data set of the woman, by a parameter providing module,
- providing an artificial intelligence by an artificial intelligence providing module, which has been trained to provide, as an output, a temporal breast cancer risk being indicative of a risk of detecting cancer in a breast of a woman within a predetermined time interval after a negative breast cancer detection result obtained by carrying out a breast cancer detection procedure defined by the breast cancer detection parameter, wherein the artificial intelligence has been provided, as an input, with a) a probability parameter, forthe woman, being indicative of a probability of having breast cancer and b) a density parameter being indicative of a density-related property of breast tissue of a breast of the woman, wherein the probability parameter and the density parameter have been determined based on a breast image data set of the woman,
- determining the temporal breast cancer risk based on the provided probability parameter and the provided density parameter by using the provided artificial intelligence by a temporal breast cancer risk determination module.

In a further aspect of the present invention a training method for training an artificial intelligence to be used by an apparatus for determining a temporal breast cancer risk as defined by claim 1 is presented, wherein the training method comprises:
- providing training data sets by a training data sets providing unit, wherein each training data set includes, as input data, i) a probability parameter, for a woman, being indicative of a probability of having breast cancer and ii) a density parameter being indicative of a density-related property of breast tissue of a breast of the woman, wherein the probability parameter and the density parameter have been determined based on a breast image data set of the woman, and, as output data, a temporal breast cancer risk being indicative of a risk of detecting cancer in a breast within a predetermined time interval after a negative breast cancer detection result obtained by carrying out a breast cancer detection procedure defined by the breast cancer detection parameter,
- providing an artificial intelligence to be trained by an artificial intelligence providing module,
- training the provided artificial intelligence by a training module by using the provided training data sets such that deviations between i) outputs of the artificial intelligence, which are output by the artificial intelligence if the inputs of the training data sets are input into the artificial intelligence, and ii) outputs of the training data sets are minimized.

In another aspect of the present invention a computer program for determining a temporal breast cancer risk being indicative of detecting cancer in a breast of a woman within a predetermined time interval, after a negative breast cancer detection result obtained by carrying out a breast cancer detection procedure defined by a breast cancer detection parameter, is presented, wherein the computer program comprises program code means for causing a computer to carry out the steps of the method for determining a temporal breast cancer risk as defined by claim 12, when the computer program is run on the computer.

In a further aspect of the present invention a computer program for training an artificial intelligence to be used by an apparatus for determining a temporal breast cancer risk as defined by claim 1 is presented, wherein the computer program comprises program code means for causing a computer to carry out the steps of the training method as defined in claim 13, when the computer program is run on the computer.

It shall be understood that the apparatus for determining a temporal breast cancer risk of claim 1, the training apparatus of claim 11, the method for determining a temporal breast cancer risk of claim 12, the training method of claim 13, the computer program for determining a temporal breast cancer risk of claim 14, and the training computer program of claim 15, have similar and/or identical preferred embodiments, particularly as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
- Fig. 1: shows schematically and exemplarily an embodiment of an apparatus for determining a temporal breast cancer risk,
- Fig. 2: illustrates schematically and exemplarily illustrates the determination of the temporal breast cancer risk,
- Fig. 3: shows schematically and exemplarily an embodiment of a training apparatus for training an artificial intelligence to be used by the apparatus shown in Fig. 1,
- Fig. 4: shows a flowchart exemplarily illustrating a method for determining a temporal breast cancer risk,
- Fig. 5: shows a flowchart exemplarily illustrating a training method for training an artificial intelligence to be used by the apparatus shown in Fig. 1, and
- Fig. 6: exemplarily shows images of breasts having different amounts and distributions of dense breast tissue.

### DETAILED DESCRIPTION OF EMBODIMENTS

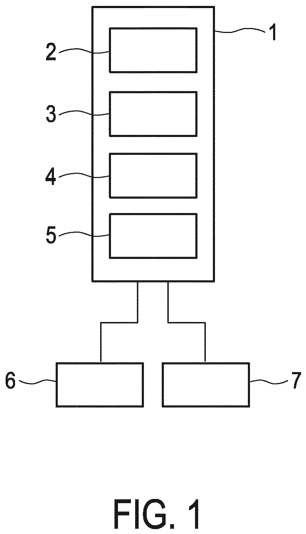

Fig. 1 shows schematically and exemplarily an embodiment of an apparatus for determining a temporal breast cancer risk being indicative of detecting cancer in a breast of a woman within a predetermined time interval after a negative breast cancer detection result obtained by carrying out a breast cancer detection procedure defined by a breast cancer detection parameter. The apparatus 1 comprises a breast image data set providing module 2 configured to provide a breast image data set of a woman. The breast image data set can show a single breast or both breasts of the woman, wherein in this embodiment the breast image data set includes a DPT image and a DM image of both breasts.

The breast image data set has been acquired, in order to determine whether the woman has breast cancer, wherein in this case breast cancer could fortunately not be detected in the breast image data set. The breast image data set has been acquired during a breast cancer screening appointment, wherein the breast cancer screening and hence the acquisition of the breast image data set is repeated in certain time intervals.

In this embodiment the breast image data set providing module 2 is a storage in which the breast image data set is stored and from which it can be retrieved for providing the same. However, the breast image data set providing module could also be a receiving module for receiving the breast image data set from another device like a breast image data set acquisition device and for providing the received breast image data set. The breast image data set providing module can also be the breast image data set acquisition device itself.

The apparatus 1 further comprises a parameter providing module 3 configured to provide a probability parameter, for the woman, being indicative of a probability of having breast cancer and to provide a density parameter being indicative of a density-related property of breast tissue of a breast of the woman, wherein the probability parameter and the density parameter have been determined based on the provided breast image data set of the woman. In this embodiment the parameter providing module 3 is configured to actually determine the probability parameter and the density parameter based on the breast image data set provided by the breast image data set providing module 2. For determining the probability parameter based on the provided breast image data set known modules and techniques can be used, which might utilize artificial intelligence for determining the probability parameter. For instance, the Transpara module of the company ScreenPoint Medical can be used to determine the probability parameter. However, it is also possible to use another module or technique for determining the probability parameter based on the provided breast image data set. For instance, the technique disclosed in the article "An interpretable classifier for high-resolution breast cancer screening images utilizing weakly supervised localization" by Y. Shen etal., Medical Image Analysis, volume 68, 101908, ISSN 1361-8415, (2021) and/or the technique disclosed in the article "International evaluation of an AI system for breast cancer screening" by S. M. McKinney et al., Nature, volume 577, pages 89 to 94, ISSN 0028-0836 (2020), which are herewith incorporated by reference.

In this embodiment the density parameter is a dense density parameter being indicative of an amount of dense breast tissue of the breast having a density being larger than the density of other breast tissue of the breast. In particular, in this embodiment the dense density parameter is indicative of the amount of glandular and connective tissue, which has a density being larger than the density of the other tissue of the breast, i.e. the fat tissue. The amount is preferentially a relative amount, i.e. the dense density parameter can indicate the amount of the glandular and connective tissue of the breast relative to the entire breast tissue of the breast. The amount hereby preferentially refers to the amount in volume or area. For determining the dense density parameter known techniques can be used like the LIBRA software or the technique disclosed in the above mentioned article by van Engeland et al.

In this embodiment the parameter providing module 3 is further configured to provide a density-distribution-based breast cancer risk, wherein the density-distribution-based breast cancer risk has been determined based on a density distribution parameter which is indicative of the density distribution in the breast tissue and which has been determined based on the breast image data set of the breast. The parameter providing module 3 is configured to actually determine the density distribution parameter based on the provided breast image data set of the breast and to determine the density-distribution-based breast cancer risk based on the determined density distribution parameter. For determining the density distribution parameter based on the provided breast image data set and for determining the density-distribution-based breast cancer risk based on the determined density distribution parameter known modules and techniques can be used like the technique disclosed in the above mentioned article by M. Kallenberg et al.

The density distribution parameter is preferentially indicative of the distribution of glandular and connective tissue within the respective breast, wherein the density distribution parameter is indicative of at least one characteristic of this distribution like its degree of homogeneity, its degree of cloudiness, the amount and length of linear structures, et cetera. In an embodiment, the techniques disclosed in the above mentioned article by K. Holland et al. could be used for determining the density distribution parameter. However, also other techniques could be used for determining the density distribution parameter and also the density-distribution-based breast cancer risk like techniques which are based on the grey-level coocurrence matrix method. For instance, the techniques disclosed in the article "A novel and fully automated mammographic texture analysis for risk prediction: results from two case-control studies" by C. Wang et al., Breast Cancer Research, volume 19, article number 114 (2017), which is herewith incorporated by reference, could be used for determining the density distribution parameter and the density-distribution-based breast cancer risk.

Although the parameter providing module 3 is configured to determine the above mentioned parameters based on the provided breast image data set, in another embodiment the parameter providing module can also just be a receiving module configured to receive the respective parameter from another module which determines the respective parameter and to provide the received respective parameter. The parameter providing module can also be a storage in which a determined parameter is stored and from which it is retrieved for providing the stored parameter.

The apparatus 1 further comprises an artificial intelligence providing module 4 configured to provide an artificial intelligence which has been trained to provide, as an output, a temporal breast cancer risk being indicative of a risk of detecting cancer in a breast of a woman within a predetermined time interval after a negative breast cancer detection result obtained by carrying out a breast cancer detection procedure defined by the breast cancer detection parameter, wherein this output is provided, after the artificial intelligence has been provided, as an input, with a) a probability parameter, for the woman, being indicative of a probability of having breast cancer and b) a density parameter being indicative of a density-related property of breast tissue of a breast of the woman, wherein the probability parameter and the density parameter have been determined based on a breast image data set of the woman. The provided artificial intelligence has been trained such that it receives as an input exactly the type of probability parameter and the type of density parameter which are provided by the parameter providing module 3. The artificial intelligence is, for instance, a trained decision tree or a trained neural network. However, the artificial intelligence can also be another kind of artificial intelligence, wherein preferentially it is not a convolutional neural network and further preferred not a deep convolutional neural network. Besides a neural network or a decision tree, the artificial intelligence could also be based on a statistical machine learning method such as kernel density estimation or Gaussian mixture modeling.

Since in this embodiment also the density-distribution-based breast cancer risk is considered, the provided artificial intelligence has been trained to provide, as the output, the temporal breast cancer risk, after the artificial intelligence has been provided, as the input, also with a density-distribution-based breast cancer risk, wherein the density-distribution-based breast cancer risk has been determined based on a density distribution parameter which is indicative of the density distribution in the breast and which has been determined based on a breast image data set of the breast.

The apparatus 1 further comprises a temporal breast cancer risk determination module 5 configured to determine the temporal breast cancer risk based on the provided probability parameter, the provided density parameter and in this embodiment also the provided density-distribution-based breast cancer risk by using the provided artificial intelligence.

The determined temporal breast cancer risk can finally be shown on an output unit 7 like a display. The apparatus 1 further comprises an input unit like a keyboard, a computer mouse, a touchpad, et cetera, in order to allow a user to interact with the apparatus 1 and to provide, for instance, further parameters to be considered by the temporal breast cancer risk determination module 5 like the age of the respective woman, the personal history, genetic information, the body mass index et cetera. This additional information can also be provided via another means like another device in which this information is stored. If this additional information is used for determining the temporal breast cancer risk, the provided artificial intelligence is preferentially trained to also consider this additional information as input, i.e. the training data sets then also include this additional information as input. This additional information is preferentially information, which is not image based, and which influences the temporal breast cancer risk. This additional information could hence also be regarded as defining non-imaging risk factors.

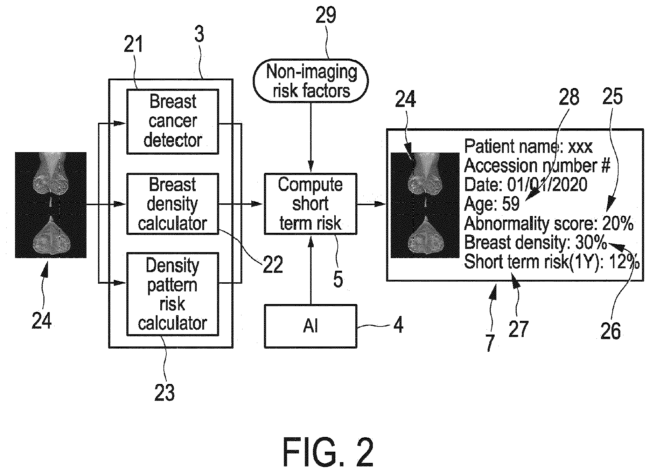

Fig. 2 schematically and exemplarily illustrates the determination of the temporal breast cancer risk.

As illustrated in Fig. 2, the breast image data set 24 is provided to a breast cancer detector 21 of the parameter providing module 3, wherein the breast cancer detector 21 is the part of the parameter providing module 3, which determines the probability parameter being indicative of the probability of having breast cancer. The breast cancer detector 21 is hence, for instance, a Transpara module of the company ScreenPoint Medical.

In this example the parameter providing module 3 also comprises a breast density calculator 22 being the part of the parameter providing module 3 which determines the density parameter being, in this embodiment, the above mentioned dense density parameter defining the percentage of the entire breast having the dense breast tissue, i.e. having the glandular and connective breast tissue, wherein this percentage is calculated as a volume percentage or an area percentage.

The parameter providing module 3 further comprises, in this example, a density pattern risk calculator 23 being the part of the parameter providing module 3, which determines the density distribution parameter and determines the density-distribution-based breast cancer risk based on the determined density distribution parameter. As explained above, the density distribution parameter is determined based on the provided image data set 24.

The temporal breast cancer risk determination module 5 then determines the temporal breast cancer risk, which might also be regarded as being a short term risk, based on the determined probability parameter, the determined density parameter and the determined density-distribution-based breast cancer risk. Also a non-imaging risk factor 29 like the age might be used for determining the temporal breast cancer risk.

The determined temporal breast cancer risk is finally shown on the display 7. In this embodiment the determined temporal breast cancer risk is the risk of detecting cancer in a breast of a woman within one year after a negative breast cancer detection result. In Fig. 2 this short term risk is 12% (reference sign 27). Moreover, in this example the dense density parameter, which is named "breast density" in Fig. 2, is 30% (reference sign 26). The display 7 also shows the age of the woman (reference sign 28). In Fig. 2 also the probability parameter is shown, wherein in this figure this probability parameter is named "abnormality score" (reference sign 25).

In this embodiment the artificial intelligence proving module 4 is a storage in which the trained artificial intelligence is stored and from which it is retrieved for providing the same. However, in another embodiment the artificial intelligence providing module can also be a receiving module for receiving the trained artificial intelligence from another device and for providing the received artificial intelligence. The artificial intelligence providing module can also be configured to train the artificial intelligence as it will be described further below with respect to the training apparatus. In particular, the artificial intelligence providing module can be configured to further train an already trained artificial intelligence.

The artificial intelligence providing module 4 can be configured to provide the artificial intelligence such that it is trained to provide a single temporal breast cancer risk for a single predetermined time interval or several temporal breast cancer risks for several predetermined time intervals. In particular, the artificial intelligence providing module 4 can be configured to provide the artificial intelligence such that it is trained to provide, as an output, temporal breast cancer risks for different predetermined time intervals, wherein a respective temporal breast cancer risk is indicative of a risk of detecting cancer in the breast within a respective predetermined time interval after a negative breast cancer detection result obtained by carrying out a breast cancer detection procedure defined by the breast cancer detection parameter, wherein the artificial intelligence has been provided, as the input, with the types of parameters which should finally be used to determine the temporal breast cancer risks. In this embodiment these parameters are the probability parameter, the dense density parameter and the density-distribution-based-breast cancer risk. The temporal breast cancer risk determination module is then configured to determine the temporal breast cancer risks for the different predetermined time intervals based on the provided probability parameter, the provided dense density parameter and the provided density-distribution-based breast cancer risk.

The artificial intelligence providing module 4 can also be configured to provide the artificial intelligence such that it is trained to provide, as an output, temporal breast cancer risks for different types of cancer, wherein a respective temporal breast cancer risk is indicative of a risk of detecting cancer of the respective cancer type in the breast within the predetermined time interval after a negative breast cancer detection result obtained by carrying out a breast cancer detection procedure defined by the breast cancer detection parameter, wherein the artificial intelligence has been provided, as the input, with the types of parameters which should finally be used for determining the temporal breast cancer risk being, in this embodiment, the probability parameter, the dense density parameter and the density-distribution-based breast cancer risk. In this case, the temporal breast cancer risk determination module 5 is configured to determine the temporal breast cancer risks forthe different cancer types based on the provided probability parameter, the provided dense density parameter and the provided density-distribution-based breast cancer risk by using the provided artificial intelligence. The parameter providing module 3 is therefore, in this example, configured to provide not a single probability parameter for a respective woman, but to provide different probability parameters for the different cancer types being indicative of a probability of having breast cancer of a respective cancer type. The artificial intelligence providing module 4 can be configured to provide the artificial intelligence such that it is trained to provide, as an output, temporal breast cancer risks for different types of cancer, wherein a respective temporal breast cancer risk is indicative of a risk of detecting cancer of the respective cancer type in the breast within the predetermined time interval after a negative breast cancer detection result obtained by carrying out a breast cancer detection procedure defined by the breast cancer detection parameter, wherein the artificial intelligence has been provided, as the input, with different probability parameters forthe different cancertypes being indicative of a probability of having breast cancer of a respective cancer type, the dense density parameter and the density-distribution-based breast cancer risk. The temporal breast cancer with determination module 5 can be configured to determine the temporal breast cancer risks for the different cancer types based on the provided probability parameters, the provided dense density parameter and the provided density-distribution-based breast cancer risk.

In an embodiment, the temporal breast cancer risks are determined for different predetermined times and also for the different cancer types. It can therefore be determined, for instance, the likelihood of detecting cancer within one year, within two years, within three years, et cetera for the different cancer types.

The apparatus 1 can be configured to determine the temporal breast cancer risk per breast of a woman or for both breasts of the woman, wherein the latter refers to a risk that in any of the two breasts cancer will be detected in the predetermined time interval. In particular, the parameter providing module 3 can be configured to provide a respective probability parameter, a respective density parameter being, in this embodiment, a dense density parameter, and in this embodiment also the density-distribution-based breast cancer risk for each breast of the woman, wherein the respective probability parameter, the respective density parameter and the respective density-distribution-based breast cancer risk have been determined based on the provided breast image data set 24 showing both breasts of the woman. In this case the artificial intelligence providing module 4 can be configured to provide, as the artificial intelligence, an artificial intelligence which has been trained to provide, as an output, i) a respective temporal breast cancer risk being indicative of a risk of detecting cancer in a respective breast of a woman within a predetermined time interval after a negative breast cancer detection result obtained by carrying out a breast cancer detection procedure defined by the breast cancer detection parameter and/or ii) a common temporal breast cancer risk being indicative of a risk of detecting cancer in any of the two breasts of a woman within the predetermined time interval after a negative breast cancer detection result obtained by carrying out a breast cancer detection procedure defined by the breast cancer detection parameter, wherein the artificial intelligence has been provided, as an input, with a) a respective probability parameters being indicative of a respective probability of having breast cancer in a respective breast of the woman, b) a respective density parameter being indicative of, in this embodiment, an amount of glandular and connective tissue of the respective breast relative to the entire respective breast and in this embodiment c) a respective density-distribution-based breast cancer risk for the respective breast of the woman, wherein these parameters and the density-distribution-based breast cancer risk have been determined based on a breast image data set of the woman. The temporal breast cancer risk determination module 5 can then be configured to determine the temporal breast cancer risk as i) a respective temporal breast cancer risk being indicative of a risk of detecting cancer in the respective breast of the woman within the predetermined time interval after the negative breast cancer detection result obtained by carrying out a breast cancer detection procedure defined by the breast cancer detection parameter and/or ii) a common temporal breast cancer risk being indicative of a risk of detecting cancer in any of the two breasts of the woman within the predetermined time interval after the negative breast cancer detection result, obtained by carrying out a breast cancer detection procedure defined by the breast cancer detection parameter, based on the probability and density parameters and the density-distribution-based breast cancer risks, which have been provided by the parameter providing module 3, by using the artificial intelligence provided by the artificial intelligence providing module 4.

The artificial intelligence providing module 4 and the temporal breast cancer risk determination module 5 are preferentially configured such that the determined temporal breast cancer risk is a relative risk. This means that it is a risk of detecting breast cancer for the respective woman within the predetermined time interval after a negative breast cancer detection result, obtained by carrying out a breast cancer detection procedure defined by the breast cancer detection parameter, relative to the risk of detecting breast cancer for a reference group of women within the predetermined time interval after a negative breast cancer detection result obtained by carrying out a breast cancer detection procedure defined by the breast cancer detection parameter. This reference group of women is a group only comprising women which had a negative breast cancer detection result.

In an embodiment the artificial intelligence providing module 4 is configured to have several artificial intelligences for different breast cancer detection parameters and hence for different breast cancer detection procedures, wherein the apparatus 1 further comprises an input unit 6 for allowing a user to indicate a certain breast cancer detection parameter and hence a certain breast cancer detection procedure. The artificial intelligence providing module 4 is configured to provide the artificial intelligence which corresponds to the indicated certain breast cancer detection procedure. Thus, a user can adapt the artificial intelligence and hence the temporal breast cancer risk determination to the respective breast cancer detection procedure which led to the current negative breast cancer detection result.

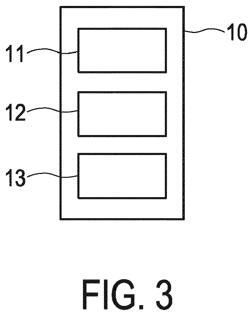

Fig. 3 shows schematically and exemplarily an embodiment of a training apparatus for training an artificial intelligence to be used by the above described apparatus 1 for determining the temporal breast cancer risk.

The training apparatus 10 comprises a training data sets providing unit 11 configured to provide training data sets, wherein each training data set includes, as input data, i) a probability parameter, for a woman, being indicative of a probability of having breast cancer, ii) a density parameter being indicative of a density-related property of breast tissue of a breast of the woman and being, in this embodiment, a dense density parameter, and iii) in this embodiment a density-distribution-based breast cancer risk, wherein the probability parameter and the density parameter have been determined based on a breast image data set of the woman and the density-distribution-based breast cancer risk has been determined based on a density distribution parameter which is indicative of the density distribution in the breast and which has been determined based on the breast image data set of the breast. Each training data set includes, as output data, a temporal breast cancer risk being indicative of a risk of the detecting cancer in a breast within a predetermined time period after a negative breast cancer detection result obtained by carrying out a breast cancer detection procedure defined by the breast cancer detection parameter.

The apparatus 10 further comprises an artificial intelligence providing module 12 configured to provide an artificial intelligence to be trained. This provided artificial intelligence can be an initial artificial intelligence which has not been trained before or it can be an already trained artificial intelligence, wherein the trained artificial intelligence should be improved due to the training.

The training apparatus 10 also comprises a training module 13 configured to train the provided artificial intelligence by using the provided training data sets such that deviations between i) outputs of the artificial intelligence, which are output by the artificial intelligence if the inputs of the training data sets are input into the artificial intelligence, and ii) outputs of the training data sets are minimized. For calculating the deviation to be minimized known deviation measures can be used like a least squares measure.

In this embodiment, the training data sets are determined based on breast image data sets of breasts of woman, in which breast cancer was not identified at screening, i.e. for which a negative breast cancer detection result was obtained by carrying out a breast cancer detection procedure defined by the breast cancer detection parameter, and in which breast cancer was detected within the predetermined time interval after screening, for instance, within one ortwo years after screening. The breast image data sets, which are preferentially used for determining the training data sets, are acquired during the screening. Preferentially, for determining the training data sets also breast image data sets of breasts of women are used, in which breast cancer was not detected at screening and in which breast cancer was also not detected within the predetermined time interval after screening. The breast image data sets can be used for determining the training input data, i.e., in this embodiment, the probability parameter, the density parameter and the density-distribution-based breast cancer risk, and the training output data, i.e. the temporal breast cancer risks.

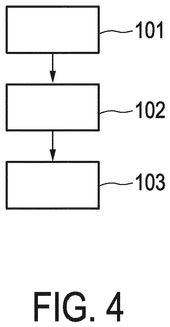

In the following an embodiment of a method for determining a temporal breast cancer risk being indicative of detecting cancer in a breast of a woman within a predetermined time interval after a negative breast cancer detection result obtained by carrying out a breast cancer detection procedure defined by a breast cancer detection parameter will be exemplarily described with reference to a flowchart shown in Fig. 4.

In step 101 a probability parameter, for the woman, being indicative of a probability of having breast cancer and a density parameter being indicative of a density-related property of breast tissue of a breast of the woman are provided. The probability parameter and the density parameter have been determined based on a breast image data set of the woman. In this embodiment the density-related property is a dense density property and, in step 101, also a density distribution-based breast cancer risk is provided as a further parameter.

In step 102 an artificial intelligence is provided. The artificial intelligence has been trained to provide, as an output, a temporal breast cancer risk being indicative of a risk of detecting cancer in a breast of a woman within a predetermined time interval after a negative breast cancer detection result obtained by carrying out a breast cancer detection procedure defined by the breast cancer detection parameter, wherein the artificial intelligence has been provided, as an input, with a) a probability parameter, for the woman, being indicative of a probability of having breast cancer, b) a density parameter being indicative of a density-related property of breast tissue of a breast of the woman, which is, in this embodiment, a dense density parameter, and in this embodiment c) a density-distribution-based breast cancer risk. The probability parameter and the density parameter used for training the artificial intelligence have been determined based on a breast image data set of the woman. Also the density-distribution-based breast cancer risk has been determined based on the breast image data set of the woman.

In step 103, the temporal breast cancer risk is determined based on the provided probability parameter, the provided density parameter and, in this embodiment, the provided density-distribution-based breast cancer risk by using the provided artificial intelligence.

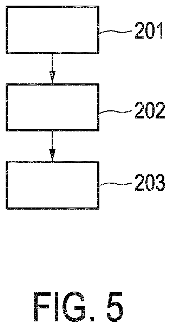

In the following an embodiment of a training method for training an artificial intelligence to be used by the apparatus for determining a temporal breast cancer risk will be exemplarily described with reference to a flowchart shown in Fig. 5.

In step 201 training data sets are provided. Each training data set includes, as input data, i) a probability parameter, for a woman, being indicative of a probability of having breast cancer, ii) a density parameter being indicative of a density-related property of breast tissue of a breast of the woman, wherein the probability parameter and the density parameter have been determined based on a breast image data set of the woman. In this embodiment the density parameter is a dense density parameter and each training data set also includes, as a further input, a density-distribution-based breast cancer risk. This density-distribution-based breast cancer risk has been determined based on a density distribution parameter which has been determined based on the breast image data set. Moreover, each training data set comprises, as output data, a temporal breast cancer risk being indicative of a risk of detected cancer in a breast within a predetermined time interval after a negative breast cancer detection result obtained by carrying out a breast cancer detection procedure defined by the breast cancer detection parameter.

In step 202 an artificial intelligence to be trained is provided, and, in step 203, the provided artificial intelligence is trained by using the provided training data sets such that deviations between i) outputs of the artificial intelligence, which are output by the artificial intelligence if the inputs of the training data sets are input into the artificial intelligence, and ii) outputs of the training data sets are minimized.

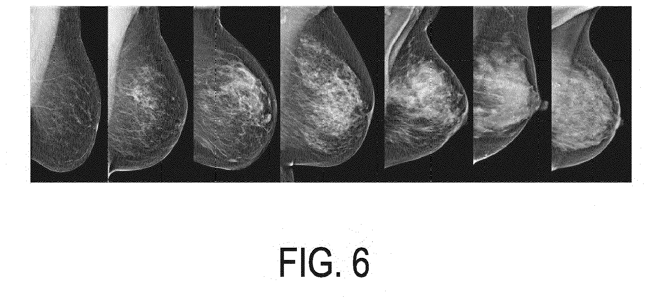

The sensitivity of mammographic screening can be seriously impaired in women with dense breast tissue. This reduced sensitivity can be due to the relatively dense glandular and connective breast tissues having the same x-ray attenuation properties as tumors and thus both showing equally bright on mammographic images. Fig. 6 schematically and exemplarily shows mammographic images, wherein the dense breast tissue, i.e. its amount and also its distribution and hence corresponding dense breast tissue patterns, can very well be seen. In Fig. 6 the overall density of the breast tissue increases from left to right, i.e. Fig. 6 shows seven images of seven different breasts with increasing overall density from left to right.

The dense breast tissue can mask tumors for radiologists such that breast cancer might remain undetected. As approximately 35 to 40% of the screening population has dense breasts, this masking effect has a strong impact and limits effectiveness of breast cancer screening. The likelihood that breast cancer is not detected during a breast cancer detection examination, although it is in fact present, therefore depends on the density of the breast tissue. The temporal breast cancer risk determination module with the artificial intelligence is therefore configured to determine the temporal breast cancer risk, which is indicative of detecting cancer in a breast of a woman within a predetermined time interval after a negative breast cancer detection result obtained by carrying out a breast cancer detection procedure defined by the breast cancer detection parameter, by considering a density parameter being indicative of a density-related property of the breast tissue. It has been found that already this density parameter together with a probability parameter being indicative of a probability of having breast cancer allows to very reliably determine the temporal breast cancer risk.

Based on the determined temporal breast cancer risk, further future breast cancer detection measurements can be planned. For instance, an already planned breast cancer screening interval can be increased or decreased. Also a more sensitive breast cancer detection measurement can be carried out like MRI. Thus, a supplemental screening examination might be carried out, which could also be based on ultrasound imaging.

The temporal breast cancer risk determination module can also be configured to suggest a supplemental breast cancer detection measurement or a modification of a breast cancer screening interval based on predetermined rules and based on the determined temporal breast cancer risk, wherein the rules define whether a supplemental breast cancer detection measurement should be carried out now and, if so, which supplemental breast cancer detection measurement should be carried out, and/or whether a breast cancer screening interval should be modified at least based on the determined temporal breast cancer risk. The rules can be configured to consider further parameters like the age of the respective woman, genetic information for the woman, personal history, body mass index, et cetera. The apparatus can also comprise a further module, which could be named suggestion module, which carries out this determination based on the rules and based at least on the determined temporal breast cancer risk. The apparatus is preferentially adapted to allow a user to modify the rules. For instance, the temporal breast cancer determination module or the recommendation module might provide a user interface for allowing a user to modify the rules via the input unit 6.

Although in above described embodiments the temporal breast cancer risk determination module and the artificial intelligence are such that the temporal breast cancer risk is determined based on certain parameters like the dense density parameter and the probability parameter, also other parameters can be used for determining the temporal breast cancer risk. For instance, as an alternative or in addition to the dense density parameter, a dense distribution parameter being indicative of the density distribution in the breast can be used for determining the temporal breast cancer risk by utilizing a correspondingly trained artificial intelligence. Also a parameter extracted from a dense breast tissue thickness map and/or other information like the age, lifestyle, personal history, genetic factors, et cetera can be used for determining the temporal breast cancer risk, wherein the artificial intelligence is then trained by using training data sets which, as input data, also include this additional information. Even if further parameters and further information is used for determining the temporal breast cancer risk, preferentially only a single indicator, i.e. the determined temporal breast cancer risk, is provided, wherein really a single temporal breast cancer risk or a single temporal breast cancer risk per type of cancer and/or per predetermined time interval is preferentially provided.

The temporal breast cancer risk determination utilizing the artificial intelligence treats the density parameter as an independent factor, although the density parameter has at least dual roles, i.e. it is a strong indicator for lifetime risk and a factor that has a strong impact on sensitivity of breast cancer detection. The density parameter is considered as an independent factor when training the artificial intelligence and when determining the temporal breast cancer risk by using the trained artificial intelligence. This leads to a simpler training of the artificial intelligence, wherein nevertheless a high quality temporal breast cancer risk can be determined.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the provision of the artificial intelligence, the provision of parameters, the determination of the parameters, the determination of the temporal breast cancer risk, et cetera performed by one or several modules, units or devices can be performed by any other number of modules, units or devices. For example, steps 101 to 103 can be performed by a single unit or by any other number of different units. These procedures and/or the control of the apparatus for determining a temporal breast cancer risk and/orthe training apparatus in accordance with the above described method for determining a temporal breast cancer risk and/or training method, respectively, can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to an apparatus for determining a temporal breast cancer risk. This risk is indicative of detecting cancer in a breast within a predetermined time interval after a negative breast cancer detection result that has been obtained by carrying out a breast cancer detection procedure defined by a breast cancer detection parameter. The temporal breast cancer risk is determined based on a) a probability parameter being indicative of a probability of having breast cancer and b) a density parameter being indicative of a density-related property of breast tissue of a breast, wherein the probability parameter and the density parameter have been determined based on a breast image data set of the woman by using an artificial intelligence. The determined risk can be used, for instance, for deciding whether a supplemental examination should be performed immediately or for defining a screening interval.

## Claims

1. An apparatus for determining a temporal breast cancer risk being indicative of detecting cancer in a breast of a woman within a predetermined time interval after a negative breast cancer detection result obtained by carrying out a breast cancer detection procedure defined by a breast cancer detection parameter, the apparatus (1) comprising:
- a parameter providing module (3) configured to provide a) a probability parameter, for the woman, being indicative of a probability of having breast cancer and b) a density parameter being indicative of a density-related property of breast tissue of a breast of the woman, wherein the probability parameter and the density parameter have been determined based on a breast image data set (24) of the woman,
- an artificial intelligence providing module (4) configured to provide an artificial intelligence which has been trained to provide, as an output, a temporal breast cancer risk being indicative of a risk of detecting cancer in a breast of a woman within a predetermined time interval after a negative breast cancer detection result obtained by carrying out a breast cancer detection procedure defined by the breast cancer detection parameter, wherein the artificial intelligence has been provided, as an input, with a) a probability parameter, for the woman, being indicative of a probability of having breast cancer and b) a density parameter being indicative of a density-related property of breast tissue of a breast of the woman, wherein the probability parameter and the density parameter have been determined based on a breast image data set of the woman,
- a temporal breast cancer risk determination module (5) configured to determine the temporal breast cancer risk based on the provided probability parameter and the provided density parameter by using the provided artificial intelligence.

2. The apparatus as defined by claim 1, wherein the parameter providing module (3) is configured to provide, as the density parameter, i) a dense density parameter being indicative of an amount of dense breast tissue of the breast having a density being larger than the density of other breast tissue of the breast, and/or ii) a density distribution parameter being indicative of the density distribution in the breast.

3. The apparatus as defined by claim 2, wherein the parameter providing module (3) is configured to provide, as the dense density parameter, a parameter being indicative of an amount of glandular and connective breast tissue of the breast as the dense breast tissue having a density being larger than the density of the other breast tissue of the breast.

4. The apparatus as defined by any of claims 1 to 3, wherein
- the parameter providing module (3) is configured to also provide a density-distribution-based breast cancer risk, wherein the density-distribution-based breast cancer risk has been determined based on a density distribution parameter which is indicative of the density distribution in the breast tissue and which has been determined based on the breast image data set (24) of the breast,
- the artificial intelligence providing module (4) is configured to provide, as the artificial intelligence, an artificial intelligence which has been trained to provide, as the output, the temporal breast cancer risk, after the artificial intelligence has also been provided, as the input, with c) a density-distribution-based breast cancer risk, wherein the density-distribution-based breast cancer risk has been determined based on a density distribution parameter which is indicative of the density distribution in the breast and which has been determined based on a breast image data set of the breast, and
- the temporal breast cancer risk determination module (5) is configured to determine the temporal breast cancer risk also based on the provided density-distribution-based breast cancer risk by using the provided artificial intelligence.

5. The apparatus as defined by any of claims 1 to 4, wherein
- the parameter providing module (3) is configured to provide at least one further parameter extracted from a dense breast tissue thickness map,
- the artificial intelligence providing module (4) is configured to provide, as the artificial intelligence, an artificial intelligence which has been trained to provide, as the output, the temporal breast cancer risk, after the artificial intelligence has also been provided, as the input, with the at least one further parameter, and
- the temporal breast cancer risk determination module (5) is configured to determine the temporal breast cancer risk also based on the provided further parameter by using the provided artificial intelligence.

6. The apparatus as defined by any of claims 1 to 5, wherein
- the artificial intelligence providing module (4) is configured to provide the artificial intelligence such that it is trained to provide, as an output, temporal breast cancer risks for different types of cancer, wherein a respective temporal breast cancer risk is indicative of a risk of detecting cancer of the respective cancer type in the breast within the predetermined time interval after a negative breast cancer detection result obtained by carrying out a breast cancer detection procedure defined by the breast cancer detection parameter, wherein the artificial intelligence has been provided, as the input, with the probability parameter and the density parameter,
- the temporal breast cancer risk determination module (5) is configured to determine the temporal breast cancer risks for the different cancer types based on the provided probability parameter and the provided density parameter by using the provided artificial intelligence.

7. The apparatus as defined by claim 6, wherein
- the parameter providing module (3) is configured to provide different probability parameters for the different cancer types being indicative of a probability of having breast cancer of a respective cancer type,
- the artificial intelligence providing module (4) is configured to provide the artificial intelligence such that it is trained to provide, as an output, temporal breast cancer risks for different types of cancer, wherein a respective temporal breast cancer risk is indicative of a risk of detecting cancer of the respective cancer type in the breast within the predetermined time interval after a negative breast cancer detection result obtained by carrying out a breast cancer detection procedure defined by the breast cancer detection parameter, wherein the artificial intelligence has been provided, as the input, with different probability parameters for the different cancer types being indicative of a probability of having breast cancer of a respective cancer type and the density parameter,
- the temporal breast cancer risk determination module (5) is configured to determine the temporal breast cancer risks for the different cancer types based on the provided probability parameters and the provided density parameter by using the provided artificial intelligence.

8. The apparatus as defined by any of claims 1 to 7, wherein
- the artificial intelligence providing module (4) is configured to provide the artificial intelligence such that it is trained to provide, as an output, temporal breast cancer risks for different predetermined time intervals, wherein a respective temporal breast cancer risk is indicative of a risk of detecting cancer in the breast within a respective predetermined time interval after a negative breast cancer detection result obtained by carrying out a breast cancer detection procedure defined by the breast cancer detection parameter, wherein the artificial intelligence has been provided, as the input, with the probability parameter and the density parameter,
- the temporal breast cancer risk determination module (5) is configured to determine the temporal breast cancer risks for the different predetermined time intervals based on the provided probability parameter and the provided density parameter by using the provided artificial intelligence.

9. The apparatus as defined by any of claims 1 to 8, wherein
- the parameter providing module (3) is configured to provide a respective probability parameter and a respective density parameter for each breast of the woman, wherein the probability parameters and the density parameters have been determined based on the breast image data set (24) which shows both breasts of the woman,
- the artificial intelligence providing module (4) is configured to provide, as the artificial intelligence, an artificial intelligence which has been trained to provide, as an output, i) a respective temporal breast cancer risk being indicative of a risk of detecting cancer in a respective breast of a woman within a predetermined time interval after a negative breast cancer detection result obtained by carrying out a breast cancer detection procedure defined by the breast cancer detection parameter and/or ii) a common temporal breast cancer risk being indicative of a risk of detecting cancer in any of the two breasts of a woman within a predetermined time interval after a negative breast cancer detection result obtained by carrying out a breast cancer detection procedure defined by the breast cancer detection parameter, wherein the artificial intelligence has been provided, as an input, with a) respective probability parameters being indicative of a respective probability of having breast cancer in a respective breast of the woman and b) respective density parameters being indicative of a density-related property of breast tissue of the respective breast of the woman, wherein the probability parameters and the density parameters have been determined based on a breast image data set of the woman,
- the temporal breast cancer risk determination module (5) is configured to determine the temporal breast cancer risk as i) a respective temporal breast cancer risk being indicative of a risk of detecting cancer in the respective breast of the woman within the predetermined time interval after the negative breast cancer detection result and/or ii) a common temporal breast cancer risk being indicative of a risk of detecting cancer in any of the two breasts of the woman within the predetermined time interval after a negative breast cancer detection result, obtained by carrying out a breast cancer detection procedure defined by the breast cancer detection parameter, based on the provided probability parameters and the provided density parameters by using the provided artificial intelligence.

10. The apparatus as defined by any of claims 1 to 9, wherein the artificial intelligence providing module (4) is configured to have several artificial intelligences for different breast cancer detection parameters and hence for different breast cancer detection procedures, wherein the apparatus comprises an input unit (6) for allowing a user to indicate a certain breast cancer detection parameter and hence a certain breast cancer detection procedure, wherein the artificial intelligence providing module (4) is configured to provide the artificial intelligence which corresponds to the indicated certain breast cancer detection procedure.

11. A training apparatus for training an artificial intelligence to be used by an apparatus for determining a temporal breast cancer risk as defined by any of claims 1 to 10, wherein the training apparatus (10) comprises:
- a training data sets providing unit (11) configured to provide training data sets, wherein each training data set includes, as input data, i) a probability parameter, for a woman, being indicative of a probability of having breast cancer and ii) a density parameter being indicative of a density-related property of breast tissue of a breast of the woman, and, as output data, a temporal breast cancer risk being indicative of a risk of detecting cancer in a breast within a predetermined time interval after a negative breast cancer detection result obtained by carrying out a breast cancer detection procedure defined by the breast cancer detection parameter,
- an artificial intelligence providing module (12) configured to provide an artificial intelligence to be trained,
- a training module (13) configured to train the provided artificial intelligence by using the provided training data sets such that deviations between i) outputs of the artificial intelligence, which are output by the artificial intelligence if the inputs of the training data sets are input into the artificial intelligence, and ii) outputs of the training data sets are minimized.

12. A method for determining a temporal breast cancer risk being indicative of detecting cancer in a breast of a woman within a predetermined time interval after a negative breast cancer detection result obtained by carrying out a breast cancer detection procedure defined by the breast cancer detection parameter, the method comprising:
- providing a) a probability parameter, for the woman, being indicative of a probability of having breast cancer and b) a density parameter being indicative of a density-related property of breast tissue of a breast of the woman, wherein the probability parameter and the density parameter have been determined based on a breast image data set (24) of the woman, by a parameter providing module (3),
- providing an artificial intelligence by an artificial intelligence providing module (4), which has been trained to provide, as an output, a temporal breast cancer risk being indicative of a risk of detecting cancer in a breast of a woman within a predetermined time interval after a negative breast cancer detection result obtained by carrying out a breast cancer detection procedure defined by the breast cancer detection parameter, wherein the artificial intelligence has been provided, as an input, with a) a probability parameter, for the woman, being indicative of a probability of having breast cancer and b) a density parameter being indicative of a density-related property of breast tissue of a breast of the woman, wherein the probability parameter and the density parameter have been determined based on a breast image data set of the woman,
- determining the temporal breast cancer risk based on the provided probability parameter and the provided density parameter by using the provided artificial intelligence by a temporal breast cancer risk determination module (5).

13. A training method for training an artificial intelligence to be used by an apparatus for determining a temporal breast cancer risk as defined by any of claims 1 to 10, wherein the training method comprises:
- providing training data sets by a training data sets providing unit (11), wherein each training data set includes, as input data, i) a probability parameter, for a woman, being indicative of a probability of having breast cancer and ii) a density parameter being indicative of a density-related property of breast tissue of a breast of the woman, and, as output data, a temporal breast cancer risk being indicative of a risk of detecting cancer in a breast within a predetermined time interval after a negative breast cancer detection result obtained by carrying out a breast cancer detection procedure defined by the breast cancer detection parameter,
- providing an artificial intelligence to be trained by an artificial intelligence providing module (12),
- training the provided artificial intelligence by a training module (13) by using the provided training data sets such that deviations between i) outputs of the artificial intelligence, which are output by the artificial intelligence if the inputs of the training data sets are input into the artificial intelligence, and ii) outputs of the training data sets are minimized.

14. A computer program for determining a temporal breast cancer risk being indicative of detecting cancer in a breast of a woman within a predetermined time interval after a negative breast cancer detection result obtained by carrying out a breast cancer detection procedure defined by a breast cancer detection parameter, the computer program comprising program code means for causing a computer to carry out the steps of the method for determining a temporal breast cancer risk as defined by claim 12, when the computer program is run on the computer.

15. A training computer program for training an artificial intelligence to be used by an apparatus for determining a temporal breast cancer risk as defined by any of claims 1 to 10, the computer program comprising program code means for causing a computerto carry out the steps of the training method as defined in claim 13, when the computer program is run on the computer.
